# FASCICULE DE BREVET EUROPEEN

(11) **EP 1 250 121 B1**
(45) Date de publication et mention de la délivrance du brevet: **19.04.2006**
(21) Numéro de dépôt: 01903884.3
(22) Date de dépôt: 08.01.2001
(51) Int. Cl.: A61K 8/63, A61Q 19/08

(54) **UTILISATION DE LA DHEA OU CERTAINES DE SES DERIVES POUR AMELIORER L'ASPECT PAPYRACE DE LA PEAU**
VERWENDUNG DES DHEAS ODER BESTIMMTE SEINER DERIVATEN ZUR VERBESSERUNG DES PAPYRUS-ÄHNLICHES AUSSEHENS DER HAUT
USE OF DEHYDROEPIANDROSTERONE AND/OR PRECURSORS OR DERIVATIVES THEREOF TO IMPROVE PAPERY ASPECT OF THE SKIN

(30) Priorité: 12.01.2000 FR 0000349
(43) Date de publication de la demande: 23.10.2002
(73) Titulaire: L'OREAL, 75008 Paris (FR)
(72) Inventeur: DE LACHARRIERE, Olivier, F-75015 Paris (FR); NOUVEAU, Stéphanie, F-92100 Boulogne (FR)
(74) Mandataire: Renard, Emmanuelle
(86) Numéro de dépôt international: PCT/FR2001/000043
(87) Numéro de publication internationale: WO 2001/051021

(56) Documents cités:
- EP-A- 0 723 775
- EP-A- 0 908 183
- WO-A-00/13661
- WO-A-02/47650
- US-A- 5 736 537

## Description

La présente invention concerne l'utilisation d'une sapogénine ou d'un extrait naturel en contenant, dans une composition cosmétique pour application topique sur la peau, pour prévenir ou réduire l'aspect papyracé de la peau, ladite sapogénine étant présente en une quantité allant de 10⁻⁶ à 10% en poids, par rapport au poids total de la composition.

Le vieillissement cutané résulte des effets sur la peau de facteurs intrinsèques et extrinsèques. Les changements de la peau résultant du vieillissement intrinsèque ou physiologique sont la conséquence d'une sénescence génétiquement programmée où interviennent des facteurs endogènes. Ce vieillissement intrinsèque provoque notamment un ralentissement du renouvellement des cellules de la peau. Histologiquement la peau est globalement amincie, tant au niveau épidermique que dermique. La densité des macromolécules fibreuses du derme (élastine et collagène) est diminuée. Au contraire, le vieillissement extrinsèque entraîne des changements histopathologiques tels qu'une excessive accumulation de matière élastique dans le derme supérieur et une dégénérescence des fibres de collagène.

Cliniquement, les signes du vieillissement se traduisent généralement par l'apparition de rides et ridules, par un relâchement des tissus cutanés et sous-cutanés, par une perte de l'élasticité cutanée et par une atonie de la texture de la peau. La perte de fermeté et de tonicité de la peau, comme les rides et les ridules, s'explique au moins en partie par une atrophie dermique et épidermique ainsi qu'un aplatissement de la formation dermoépidermique ; la peau est moins épaisse et plus flasque, et l'épaisseur de l'épiderme diminue.

En outre, le teint de la peau est généralement modifié ; il apparaît plus pâle et plus jaune ; ceci semble être dû essentiellement à une désorganisation de la microcirculation (moins d'hémoglobine au niveau du derme papillaire). Un autre signe clinique du vieillissement est l'aspect sec et rêche de la peau qui est dû essentiellement à une desquamation plus importante ; ces squames, en diffractant les rayons lumineux, participent aussi à l'aspect un peu gris du teint.

De plus, de nombreuses taches colorées et/ou plus foncées apparaissent à la surface de la peau, et plus spécialement sur les mains, conférant à la peau une hétérogénéité. En général, ces taches sont dues à une production importante de mélanine dans l'épiderme et/ou le derme de la peau. Dans certains cas, ces taches peuvent devenir cancéreuses. Par ailleurs, il peut exister sur certaines zones de la peau des irritations diffuses et parfois des télangiectasies.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

Un autre signe du vieillissement cutané, qui constitue un critère majeur de la dégradation de l'apparence de la peau, est l'accentuation de l'aspect papyracé.

L'aspect papyracé se caractérise par une modification de l'aspect visuel, ainsi que de la tenue au toucher, de la peau. Plus précisément, la peau revêt visuellement l'aspect d'un papier à cigarette lui donnant une apparence similaire à celle d'une feuille de papyrus. En outre, lorsqu'elle est légèrement pincée entre le pouce et l'index, la peau forme des plis fins, aigus et nombreux ayant l'apparence d'un papier froissé. Enfin, le toucher de la peau montre que ses parties superficielles sont comme flottantes sur les parties profondes, donnant à la peau, au stade très évolué d'aspect papyracé, l'apparence d'un papier chiffonné.

Les actifs cosmétiques ou dermatologiques reconnus comme efficaces sur certains des signes du vieillissement cutané n'ont pas nécessairement d'effet sur l'aspect papyracé de la peau. Au contraire, jusqu'à présent, le seul actif connu pour être capable de limiter cette augmentation de l'aspect papyracé est l'acide rétinoïque.

Or, l'utilisation topique de l'acide rétinoïque n'est pas sans effets secondaires. A faible concentration, il entraîne ainsi fréquemment des picotements, voire des démangeaisons ou un érythème, générant un inconfort inacceptable pour une utilisation cosmétique et conduisant parfois les patients à interrompre leur traitement dans le cas d'une utilisation thérapeutique.

Aussi, la présente invention a pour but de proposer un nouvel actif permettant d'améliorer l'aspect papyracé de la peau sans avoir les effets secondaires de l'acide rétinoïque, en particulier sans entraîner d'irritation cutanée.

La demanderesse a trouvé de manière inattendue que les sapogénines, qui sont des précurseurs de la DHEA, permettaient d'atteindre ce but.

La DHEA, ou déhydroépiandrostérone, est un stéroïde naturel produit essentiellement par les glandes corticosurrénales. Elle est connue pour ses propriétés anti-âge, liées à sa capacité à promouvoir la kératinisation de l'épiderme (JP-07 196 467) et à lutter contre l'ostéoporose (US-5,824,671), ou encore dans le traitement des peaux sèches, en raison de son aptitude à augmenter la production endogène et la sécrétion de sébum et à renforcer l'effet barrière de la peau (US-4,496,556). La DHEA est également décrite dans le traitement de l'obésité et du diabète (WO 97/13500). Il a en outre été proposé d'utiliser le sulfate de DHEA contre l'alopécie (JP-60 142 908) et pour traiter différents signes du vieillissement tels que les rides, la perte d'éclat de la peau et le relâchement cutané (EP-0 723 775).

Toutefois, il n'a encore jamais été suggéré que la DHEA et/ou l'un au moins de ses précurseurs ou dérivés pouvaient avoir un effet sur l'aspect papyracé de la peau.

En outre, la demande WO 00/13661 divulgue l'utilisation de divers phytoextraits, pouvant contenir des sapogénines, pour lutter contre les désordres cutanés liés à la ménopause. Leur concentration dans les compositions qui les véhiculent n'est toutefois pas mentionnée.
La présente invention a donc pour objet l'utilisation d'une sapogénine ou d'un extrait naturel en contenant, dans une composition cosmétique pour application topique sur la peau, pour prévenir ou réduire l'aspect papyracé de la peau, ladite sapogénine étant présente en une quantité allant de 10⁻⁶ à 10% en poids, par rapport au poids total de la composition.

Par sapogénines, on entend notamment la diosgénine (ou spirost-5-èn-3-beta-ol), l'hécogénine, le smilagénine et la sarsapogénine. Les extraits naturels en contenant sont en particulier le fenugrec et les extraits de Dioscorées telles que la racine d'igname sauvage ou Wild Yam, sans que cette liste soit limitative.

La composition contenant les sapogénines est appropriée à une utilisation topique et elle contient donc un milieu physiologiquement acceptable, c'est-à-dire compatible avec la peau.

Cette composition renferme de 10⁻⁶ à 10% en poids, avantageusement de 0,1 à 5% en poids, et mieux, environ 1% en poids de sapogénine, par rapport au poids total de la composition.

Elle peut se présenter sous toutes les formes galéniques normalement utilisées pour une application topique, notamment sous forme d'une solution aqueuse, hydroalcoolique, d'une émulsion huile-dans-eau ou eau-dans-huile ou multiple, d'un gel aqueux, ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage.

De façon connue, la composition selon l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans des vésicules lipidiques et/ou dans des nanoparticules.

Bien entendu, l'homme du métier veillera à choisir ces éventuels composés complémentaires, actifs ou non actifs, et/ou leur quantité, de telle manière que les propriétés avantageuses des sapogénines ne soient pas, ou sensiblement pas, altérées par l'adjonction envisagée.

Lorsque la composition selon l'invention est une émulsion, la proportion de la phase grasse peut aller de 0,5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20% en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les agents kératolytiques et/ou desquamants, les agents dépigmentants, les filtres UV, les agents anti-radicaux libres et leurs mélanges. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des compositions pour le bain, des fonds de teint et des crèmes teintées. Dans ces derniers cas, la composition contient des pigments.

L'invention va maintenant être illustrée à l'aide des exemples non limitatifs suivants.

### Exemple 1 : Mise en évidence de l'effet de la DHEA sur l'aspect papyracé

### a- Protocole

On a testé une émulsion huile-dans-eau gélifiée, renfermant 1% en poids de DHEA comme seul actif, par comparaison avec un véhicule constitué d'une émulsion identique mais ne renfermant pas de DHEA.

Les produits ont été testés sur un groupe de quarante sujets de sexe féminin, âgés de 55 à 70 ans. La moitié des sujets a appliqué le véhicule et l'autre moitié a appliqué l'émulsion à base de DHEA, le choix des sujets étant effectué selon une randomisation générée par informatique. L'émulsion a été appliquée matin et soir pendant quatre mois sur le dos d'une seule main, l'autre main servant de témoin. Les quantités appliquées ont été contrôlées par pesée des tubes au début et à la fin de l'essai.

L'aspect papyracé est évalué cliniquement en aveugle sur le dos de chaque main, au début de l'essai et après quatre mois, par le même clinicien. L'aspect papyracé est représenté par un score clinique variant de 0 (aspect papyracé absent ou très peu marqué) à 9 (aspect papyracé très marqué).

L'analyse statistique est réalisée par des tests de Wilcoxon.

### b- Résultats

Les résultats sont illustrés par la figure 1 présentée en annexe.

Cette figure montre qu'au temps initial (To), les différences de scores de l'aspect papyracé entre les deux mains sont quasi nulles, quel que soit le groupe de sujets considéré.

Après quatre mois de traitement, cette différence reste quasi-nulle, en tout cas non significative, pour le groupe traité avec le véhicule (placebo). Au contraire, pour le groupe traité avec la DHEA, on observe une différence de scores entre les deux mains : le score de l'aspect papyracé est plus faible sur la main traitée avec la DHEA que sur la main témoin. Cette diminution est significative à p = 0,05.

On peut en déduire que l'application de DHEA par voie topique permet d'améliorer l'aspect de la peau en réduisant le score d'aspect papyracé.

### Exemple 2 : Effet de la DHEA sur l'élasticité, la texture et la flaccidité cutanées

Le but de cet exemple est de démontrer que l'amélioration de l'aspect papyracé de la peau n'est pas lié à un éventuel effet de la DHEA sur l'élasticité, la texture et/ou la flaccidité de la peau.

### a- Protocole

Ce test a été réalisé simultanément à celui décrit dans l'Exemple 1, en appliquant les mêmes produits, sur les mêmes groupes de sujets, et dans les mêmes conditions, excepté que les produits ont été appliqués sur la peau du visage.

La texture de la peau et la flaccidité ont été évaluées en aveugle par le même clinicien et sont représentées par des scores cliniques variant de 0 (paramètre peu ou pas marqué) à 9 (paramètre très marqué).

L'élasticité cutanée a été évaluée, au début de l'essai et après quatre mois, en utilisant l'appareil disponible sous la dénomination commerciale Dermal Torque Meter® auprès de DTM® Dia-Stron Limited, Andover, Hampshire, Royaume-Uni. Cet appareil permet d'étudier la réponse de la peau à une déformation appliquée dans la direction parallèle à sa surface, comme décrit dans Escoffier C et al., Age-Related Mechanical Properties of Human Skin : an In Vivo Study, J. Invest. Dermatol., 1989, 93:353-357.

L'analyse statistique a été faite par des tests de Wilcoxon.

### b- Résultats

Les résultats sont donnés dans le Tableau 1 ci-après.

**TABLEAU 1**

| PARAMETRE | DHEA | | PLACEBO | | SIGNIFICATIVITE |
|---|---|---|---|---|---|
| | T0 | T4 | T0 | T4 | |
| Texture | 4 | 3,8 | 4,05 | 3,4 | p= 0.8 |
| Flaccidité | 4,45 | 4,15 | 4,45 | 4,05 | p= 0.72 |
| Elasticité | 0,77 | 0,66 | 0,69 | 0,65 | p= 0.77 |

Il ressort du Tableau 1 ci-dessus que l'application topique de DHEA ne modifie pas significativement la texture, l'élasticité et la flaccidité de la peau. L'amélioration de l'aspect papyracé observé dans l'Exemple 1 est donc indépendant de la variation de ces paramètres.

## Revendications

1. Utilisation d'une sapogénine ou d'un extrait naturel en contenant, dans une composition cosmétique pour application topique sur la peau, pour prévenir ou réduire l'aspect papyracé de la peau, ladite sapogénine étant présente en une quantité allant de 10⁻⁶ à 10% en poids, par rapport au poids total de la composition.

2. Utilisation selon la revendication 1, **caractérisée en ce que** ladite sapogénine est choisie parmi la diosgénine, l'hécogénine, le smilagénine et la sarsapogénine.

3. Utilisation selon la revendication 1, **caractérisée en ce que** ledit extrait naturel est choisi parmi le fenugrec et les extraits de Dioscorées.

4. Utilisation selon la revendication 3, **caractérisée en ce que** ledit extrait de Dioscorée est un extrait de racine d'igname sauvage.

5. Utilisation selon l'une quelconque des revendications 1 à 4, **caractérisée en ce que** la sapogénine est présente en une quantité allant de 0,1 à 5% en poids, par rapport au poids total de la composition.

## Claims

1. Use of a sapogenin or of a natural extract containing it, in a cosmetic composition for topical application to the skin, to prevent or reduce the weathered appearance of the skin, said sapogenin being present in an amount ranging from 10⁻⁶ to 10% by weight relative to the total weight of the composition.

2. Use according to Claim 1, **characterized in that** said sapogenin is chosen from diosgenin, hecogenin, smilagenin and sarsapogenin.

3. Use according to Claim 1, **characterized in that** said natural extract is chosen from fenugrec and extracts of Dioscorea plants.

4. Use according to Claim 3, **characterized in that** said extract of Dioscorea plants is an extract of wild yam root.

5. Use according to any one of Claims 1 to 4, **characterized in that** the sapogenin is present in an amount ranging from 0.1% to 5% by weight relative to the total weight of the composition.

## Patentansprüche

1. Verwendung eines Sapogenins oder eines natürlichen Sapogenin-haltigen Extrakts in einer kosmetischen Zusammensetzung zur topischen Anwendung auf die Haut, um dem pergamentartigen Aussehen der Haut vorzubeugen oder das pergamentartige Aussehen der Haut zu vermindern, wobei das Sapogenin in einer Menge von 10⁻⁶ bis 10 Gew.-%, bezogen auf das Gesamtgewicht der Zusammensetzung, enthalten ist.

2. Verwendung nach Anspruch 1, **dadurch gekennzeichnet, dass** das Sapogenin unter Diosgenin, Hecogenin, Smilagenin und Sarsapogenin ausgewählt ist.

3. Verwendung nach Anspruch 1, **dadurch gekennzeichnet, dass** der natürliche Extrakt unter Bockshornklee und Dioscorea-Extrakten ausgewählt ist.

4. Verwendung nach Anspruch 3, **dadurch gekennzeichnet, dass** der Dioscorea-Extrakt ein Extrakt aus Wildem Yams ist.

5. Verwendung nach einem der Ansprüche 1 bis 4, **dadurch gekennzeichnet, dass** das Sapogenin in einer Menge von 0,1 bis 5 Gew.-%, bezogen auf das Gesamtgewicht der Zusammensetzung, enthalten ist.
